# EUROPEAN PATENT SPECIFICATION

(11) **EP 3 176 150 B1**
(45) Date of publication and mention of the grant of the patent: **03.06.2020**
(21) Application number: 15826489.5
(22) Date of filing: 29.07.2015
(51) Int. Cl.: A61Q 19/00, C07C 233/47, A61K 8/44

(54) **MEDIUM-CHAIN ACYL BASIC AMINO ACID DERIVATIVE**
DERIVATE VON MITTELKETTIGER ACYLBASISCHER AMINOSÄURE
DÉRIVÉ (ACYLE À CHAÎNE MOYENNE)ACIDE AMINÉ BASIQUE

(30) Priority: 30.07.2014 JP 2014154444
(43) Date of publication of application: 07.06.2017
(73) Proprietor: AJINOMOTO CO., INC., Chuo-ku Tokyo 104-8315 (JP)
(72) Inventor: KOBAYASHI, Shun, Kawasaki-shi Kanagawa 210-8681 (JP); KURAMOTO, Masayuki, Yokkaichi-shi Mie 510-0885 (JP)
(74) Representative: Mewburn Ellis LLP
(86) International application number: PCT/JP2015/071431
(87) International publication number: WO 2016/017659

(56) References cited:
- WO-A1-2013/118896
- JP-A- 2000 256 303
- JP-A- 2004 323 505
- JP-A- 2004 323 505
- SANCHEZ L ET AL: "Assessment of the potential skin irritation of lysine-derivative anionic surfactants using mouse fibroblasts and human keratinocytes as an alternative to animal testing", PHARMACEUTICAL RESE, SPRINGER NEW YORK LLC, US, vol. 21, no. 9, 1 September 2004 (2004-09-01), pages 1637-1641, XP008119242, ISSN: 0724-8741, DOI: 10.1023/B:PHAM.0000041459.63362.6F [retrieved on 2004-12-30]
- Y LIANG ET AL: "Surface Adsorption and Aggregation Properties of Novel l-Lysine-Based Gemini Surfactants", JOURNAL OF SURFACTANTS AND DETERGENTS, vol. 17, no. 4, 1 July 2014 (2014-07-01), pages 693-701, XP55426540, DE ISSN: 1097-3958, DOI: 10.1007/s11743-013-1541-6
- SUZUKI, MASAHIRO ET AL.: 'L-Lysine based gemini organogelators: their organogelation properties and thermally stable organogels' ORGANIC & BIOMOLECULAR CHEMISTRY vol. 1, no. 22, 21 November 2003, pages 4124 - 4131, XP002276454
- SUZUKI, MASAHIRO ET AL.: 'New gemini organogelators linked by oxalyl amide: organogel formation and their thermal stabilities' TETRAHEDRON LETTERS vol. 44, no. 36, 01 January 2003, pages 6841 - 6843, XP002276455 DOI: 10.1016/S0040-4039(03)01719-2
- SUZUKI, MASAHIRO ET AL.: 'Novel dumbbell-form low-molecular-weight gelators based on L- lysine: their hydrogelation and organogelation properties' NEW JOURNAL OF CHEMISTRY vol. 29, no. 11, 01 January 2005, pages 1439 - 1444, XP055346030 DOI: 10.1039/B511158G

## Description

### [Technical Field]

The present invention relates to a medium-chain acyl basic amino acid derivative that suppresses gelation, and a cosmetic composition containing same.

### [Background Art]

It has been reported that a compound represented by the following formula: 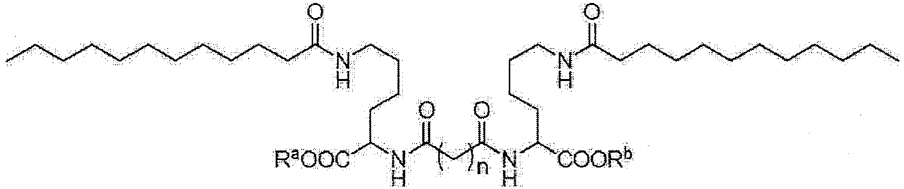 wherein R^{a} and R^{b} are each a hydrogen atom or an alkyl group, and n is an integer of 0 to 12, or a salt thereof (hereinafter to be also referred to as "lauroyl amino acid derivative") is useful for gelling or solidifying water and a liquid organic medium (patent document 1, non-patent document 1 and non-patent document 2 etc.). Because of the properties of the lauroyl amino acid derivative as a surfactant, it improves affinity to the skin when blended in a cosmetic, and is also expected to exert a moisturizing ability. Therefore, studies are ongoing as regards blending of the lauroyl amino acid derivative into cosmetics.

However, when the lauroyl amino acid derivative is blended in a liquid cosmetic such as skin lotion, skin milk and the like, a stable preparation is difficult to obtain due to the gelling ability of the lauroyl amino acid derivative per se, which problematically markedly restricts the usability thereof.

Non-patent document 3 discloses studies examining potential skin irritation of various lysine-derivative surfactants.

Non-patent document 4 discloses studies examining the surface adsorption and aggregation properties of L-lysine-based gemini surfactants including disodium(18R,23R)-12,20,21,29-tetraoxo-13,19,22,28-tetraazatetracontane-18,23-dicarboxylate, disodium (18R,25R)-12,20,23,31-tetraoxo-13,19,24,30-tetraazadotetracontane-18,25-dicarboxylate, disodium(18R,27R)-12,20,25,33-tetraoxo-13,19,26,32-tetraazatetratetracontane-18,27-dicarboxylate.

### [Document List]

### [Patent document]

patent document 1: JP-A-2004-323505

### [non-patent documents]

non-patent document 1: Org. Biomol. Chem., 2003, 1, 4124-4131
non-patent document 2: New J. Chem., 2005, 29, 1439-1444
non-patent document 3: Pharmaceutical Research, 2004, 21(9), 1637-1641
non-patent document 4: J. Surfact. Deterg., 2014, 17, 693-701

### [SUMMARY OF THE INVENTION]

### [Problems to be Solved by the Invention]

The present invention provides a medium-chain acyl basic amino acid derivative which, when blended in a liquid cosmetic such as skin lotion, skin milk and the like, suppresses gelation while maintaining affinity to the skin and the like and can afford a stable preparation.

### [Means of Solving the Problems]

The present inventors have conducted intensive studies in an attempt to solve the aforementioned problems and unexpectedly found that a medium-chain acyl basic amino acid derivative having a short acyl chain length suppresses gelation while maintaining affinity to the skin and the like, even when blended in a liquid cosmetic such as skin lotion, skin milk and the like, and the obtained preparation is stable, which resulted in the completion of the present invention.

Therefore, the present invention provides the following.
[1] A compound represented by the formula (1): 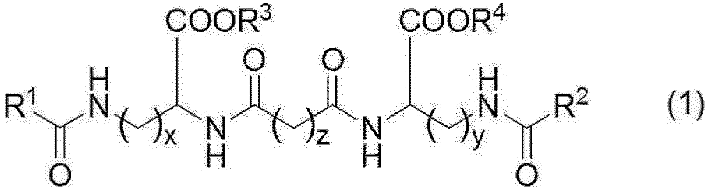 wherein
   R¹ and R² are each independently an alkyl group having 5 - 7 carbon atoms or an alkenyl group having 5 - 7 carbon atoms,
   R³ and R⁴ are each a hydrogen atom,
   z is 7 or 8, and
   x and y are each independently an integer of 2 - 4,
   or a salt thereof.
[2] The compound of [1], wherein x and y are each 4, or a salt thereof.
[3] The compound of [1] or [2], wherein R¹ and R² are each independently an alkyl group having 5 - 7 carbon atoms, or a salt thereof.
[4] A compound selected from bis(N^{ε}-octanoyl-L-lysine)sebacoylamide, bis(N^{ε}-hexanoyl-L-lysine)azelaoylamide, and bis(N^{ε}-octanoyl-L-lysine)azelaoylamide, or a salt thereof.
[5] A cosmetic composition comprising at least one kind from the compound of any of [1] - [4] and a salt thereof.

### [Effect of the Invention]

According to the present invention, a medium-chain acyl basic amino acid derivative which, when blended in a liquid cosmetic such as skin lotion, skin milk and the like, suppresses gelation while maintaining affinity to the skin and the like and can afford a stable preparation, can be provided.

### [Description of Embodiments]

The definition of each symbol of a compound represented by the formula (1) is described in the following.

R¹ and R² are each independently an alkyl group having 5 - 7 carbon atoms or an alkenyl group having 5 - 7 carbon atoms.

The alkyl group having 5 - 7 carbon atoms means a linear or branched alkyl group having 5 - 7 carbon atoms, and a pentyl group, an isopentyl group, a neopentyl group, a hexyl group, an isohexyl group, a neohexyl group, a heptyl group, an isoheptyl group, a neoheptyl group and the like can be specifically mentioned.

The alkenyl group having 5 - 7 carbon atoms means a linear or branched alkenyl group having 5 - 7 carbon atoms, and a pentenyl group, a hexenyl group, a heptenyl group and the like can be specifically mentioned.

R¹ and R² are preferably each independently an alkyl group having 5 - 7 carbon atoms, more preferably a pentyl group or a heptyl group.

R³ and R⁴ are each a hydrogen atom.

z is 7 or 8.

x and y are each independently an integer of 2 - 4.

x and y are each preferably 4.

As a compound represented by the formula (1), the following compound can be preferably mentioned.

### (compound A)

A compound wherein
R¹ and R² are each independently an alkyl group having 5 - 7 carbon atoms,
R³ and R⁴ are each a hydrogen atom,
z is 7 or 8 , and
x and y are each 4.

### (compound B)

A compound wherein
R¹ and R² are each a pentyl group or a heptyl group,
R³ and R⁴ are each a hydrogen atom,
z is 7 or 8, and
x and y are each 4.

Specific examples of a compound represented by the formula (1) include a compound selected from
bis (N^{ε}-octanoyl-L-lysine) sebacoylamide,
bis(N^{ε}-hexanoyl-L-lysine)azelaoylamide, and
bis(N^{ε}-octanoyl-L-lysine)azelaoylamide,
and a salt thereof.

Examples of a salt of the medium-chain acyl basic amino acid derivative of the present invention include alkali metal salts such as a lithium salt, a sodium salt, a potassium salt and the like; alkali earth metal salts such as a calcium salt, a magnesium salt and the like; ammonium salts; a salt of an organic amine base such as methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like, and the like.

Of these, a sodium salt or a potassium salt is preferable, and a sodium salt is more preferable.

The medium-chain acyl basic amino acid derivative of the present invention can be produced by a conventionally-used method. For example, as shown in the following formula, symmetrical medium-chain acyl basic amino acid derivative (1') can be produced by reacting N^{ω}-acyl amino acid (2) and dicarboxylic acid dichloride (3) in an appropriate solvent. 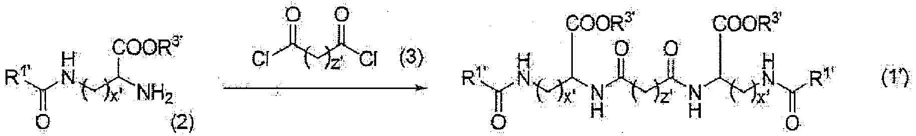 wherein R^{1'} is an alkyl group having 5 - 7 carbon atoms or an alkenyl group having 5 - 7 carbon atoms, R^{3'} is a hydrogen atom, z' is 7 or 8 , and x' is an integer of 2 - 4.

Examples of the N^{ω}-acyl amino acid (2) include N^{ε}-acyl lysine (e.g., N^{ε}-hexanoyl-L-lysine, N^{ε}-octanoyl-L-lysine etc.), N^{δ}-acyl ornithine (e.g., N^{δ}-hexanoyl-L-ornithine etc.), N^{γ}-acyl-α,γ-diaminobutyric acid and the like.

Examples of the dicarboxylic acid dichloride (3) include
azelaoyl chloride, sebacoyl chloride,
The amount of dicarboxylic acid dichloride (3) to be used is generally 0.4 - 0.6 equivalent relative to N^{ω}-acyl amino acid (2).

While the solvent is not particularly limited as long as it is inert to the reaction, examples thereof include ethers such as diethyl ether, tetrahydrofuran and the like.

In addition, asymmetric medium-chain acyl basic amino acid derivative (1") can be produced as follows. First, N^{ω}-acyl amino acid (2) and dicarboxylic acid monochloride monoester (4) are reacted in an appropriate solvent to give compound (5) (step 1). Then, the ester moiety of the obtained compound (5) is hydrolyzed in the presence of a base such as sodium hydroxide, potassium hydroxide and the like, the carboxylic acid moiety is chlorinated with a chlorinating agent such as thionyl chloride and the like, and the compound is reacted with N^{ω}-acyl amino acid (2') which is different from N^{ω}-acyl amino acid (2) used in the aforementioned step 1 (step 2), whereby derivative (1") can be produced. 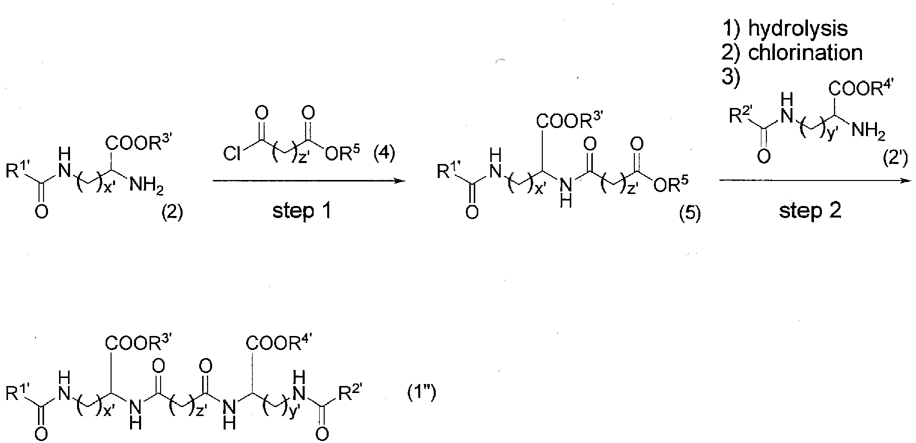 wherein R^{1'}, R^{3'}, z' and x' are as defined above, R^{2'} is an alkyl group having 5 - 7 carbon atoms or an alkenyl group having 5 - 7 carbon atoms, R^{4'} is a hydrogen atom, R⁵ is an alkyl group such as a methyl group, an ethyl group and the like, and y' is an integer of 2 - 4.

As N^{ω}-acyl amino acids (2) and (2'), N^{ω}-acyl amino acids similar to those mentioned above can be used.

As dicarboxylic acid monochloride monoester (4), a commercially available product can be used as is when it is commercially available, or one produced by a method known per se or a method analogous thereto can also be used.

A medium-chain acyl basic amino acid derivative obtained by the above-mentioned method can be converted to a salt of the medium-chain acyl basic amino acid derivative by a reaction with alkali metal hydroxide such as sodium hydroxide, potassium hydroxide and the like, alkali earth metal hydroxide such as calcium hydroxide and the like, organic amine base, or the like.

The present invention also relates to a cosmetic composition containing the above-mentioned medium-chain acyl basic amino acid derivative or a salt thereof.

The amount of the above-mentioned medium-chain acyl basic amino acid derivative or a salt thereof in the cosmetic composition of the present invention is preferably 0.01 - 10 wt%, more preferably 0.05 - 5 wt%.

Specific examples of the cosmetic composition of the present invention include facial cleanser, skin lotion, skin milk, cream, gel, beauty essence, facial mask, mask, face powder, foundation, lip rouge, cheek rouge, eyeliner, mascara, eye shadow, eyebrow pencil, shampoo, rinse, hair conditioner, hair styling agent, hair treatment and the like.

Of these, skin lotion or skin milk is preferable.

The cosmetic composition of the present invention may contain a component that can be generally added to cosmetics as long as the effect of the present invention is not inhibited. Specific examples include oil, chelating agent, surfactant, powder, amino acid, polyvalent alcohol, polyamino acid and salt thereof, water-soluble polymer, sugar alcohol and alkylene oxide adduct thereof, lower alcohol, animal and plant extract, nucleic acid, vitamin, enzyme, anti-inflammatory agent, antimicrobial agent, preservative, antioxidant, ultraviolet absorber, adiaphoretic, pigment, dye, oxidation dye, organic and inorganic powder, pH adjuster, pearly sheen agent, and wetting agent.

### [Examples]

The present invention is explained in detail in the following by referring to Examples, which are not to be construed as limitative.

The instrument used for the measurement of the compound was as described below.
¹H-NMR measurement: Bruker, AVANCE III HD NMR Spectrometer

### Production Example 1: Synthesis of bis(N^{ε}-octanoyl-L-lysine)sebacoylamide monosodium salt

N^{ε}-octanoyl-L-lysine (6.8 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was dissolved in an aqueous sodium hydroxide solution to adjust to pH 6.0, evaporated to dryness and recrystallized from methanol-diethyl ether to give the title compound (8.6 g, 94%).
¹H-NMR (400 MHz, CD₃OD, TMS, 25 °C):δ 0.89 (t, J=6.9 Hz, 6H), 1.31 (br, 28H), 1.36-1.45 (m, 4H), 1.47-1.54 (m, 4H), 1.55-1.63 (m, 8H), 1.64-1.72 (m, 2H), 1.79-1.89 (m, 2H), 2.15 (t, J=7.4 Hz, 4H), 2.23 (t, J=7.4 Hz, 4H), 3.15 (t, J=6.9 Hz, 4H), 4.35 (m, 2H)

### Production Example 2: Synthesis of bis(N^{ε}-hexanoyl-L-lysine)azelaoylamide monosodium salt

N^{ε}-hexanoyl-L-lysine (12.2 g, 50 mmol) was dissolved in water (130 g) and 25% aqueous sodium hydroxide solution (20 g), and diethyl ether (150 g) was added. Azelaoyl chloride (6.2 g, 28 mmol) was slowly added to the ether layer. The two-layer solution was stirred for about 1 hr while maintaining at 0°C, and then at room temperature for 23 hr. Then, 75% sulfuric acid was added dropwise to adjust to pH 2, and the obtained oily viscous substance was extracted with 2-propanol. 2-Propanol was evaporated under reduced pressure, the obtained compound was dissolved in an aqueous sodium hydroxide solution to adjust to pH 6.0, evaporated to dryness and recrystallized from methanol-diethyl ether to give the title compound (7.8 g, 47%).
¹H-NMR (400 MHz, CD₃OD, TMS, 25 °C):δ 0.90 (t, J=6.9 Hz, 6H), 1.31 (br, 18H), 1.36-1.45 (m, 4H), 1.47-1.54 (m, 4H), 1.55-1.63 (m, 8H), 1.64-1.72 (m, 2H), 1.79-1.89 (m, 2H), 2.15 (t, J=7.5 Hz, 4H), 2.23 (t, J=7.5 Hz, 4H), 3.15 (m, 4H₂), 4.30 (m, 2H)

### Production Example 3: Synthesis of bis(N^{ε}-octanoyl-L-lysine)azelaoylamide monosodium salt

Using N^{ε}-octanoyl-L-lysine and azelaoyl chloride, the title compound (8.0 g, 89%) was synthesized by a method almost similar to Production Example 1.
¹H-NMR (400 MHz, CD₃OD, TMS, 25 °C):δ 0.89 (t, J=6.9 Hz, 6H), 1.31 (br, 26H), 1.36-1.45 (m, 4H), 1.47-1.54 (m, 4H), 1.55-1.63 (m, 8H), 1.64-1.72 (m, 2H), 1.79-1.89 (m, 2H), 2.15 (t, J=7.4 Hz, 4H), 2.23 (t, J=7.4 Hz, 4H), 3.15 (t, J=6.9 Hz, 4H), 4.30 (m, 2H)

### Production Example 4: Synthesis of bis(N^{ε}-lauroyl-L-lysine)sebacoylamide monosodium salt

Using N^{ε}-lauroyl-L-lysine and sebacoyl chloride, the title compound was synthesized by a method almost similar to Production Example 1.

### Production Example 5: Synthesis of bis(N^{ε}-lauroyl-L-lysine)azelaoylamide monosodium salt

Using N^{ε}-lauroyl-L-lysine and azelaoyl chloride, the title compound was synthesized by a method almost similar to Production Example 1.

### Experimental Example

A cosmetic composition (skin lotion) having the composition (unit: wt%) shown in the following Table 1 was prepared by the method described in the following 1., and the stability, coatability and affinity to the skin of the obtained cosmetic composition were evaluated by the method described in the following 2. and 3.

### 1. Preparation of cosmetic composition (skin lotion)

The compound synthesized in the above-mentioned Production Example, various additives shown in the following Table 1 and water were stirred at 70°C and uniformly dissolved. The mixture was cooled to room temperature, and pH was adjusted to 6.0 with citric acid to give a cosmetic composition (skin lotion).

### 2. Evaluation of stability of cosmetic composition

The cosmetic composition prepared in the above-mentioned 1. was filled in a glass bottle, and stood at room temperature for 12 hr. Then, the glass bottle was placed upside down, visually observed, and evaluated by the following criteria.
⊙: fell when glass bottle was upside down
×: did not fall when glass bottle was upside down

### 3. Evaluation of coatability, affinity to the skin

The coatability and affinity to the skin of the cosmetic composition prepared in the above-mentioned 1. were evaluated by 5 professional panelists according to the following criteria.

### (Evaluation criteria of coatability)

When a cosmetic composition is taken on a hand from a container and actually spread on the back of the hand,
5 points: cosmetic composition can be easily taken out from the container and can be spread smoothly and uniformly
4 points: cosmetic composition can be taken out from the container and can be easily spread uniformly
3 points: cosmetic composition is rather difficult to take out from the container but can be spread uniformly
2 points: cosmetic composition is difficult to take out from the container and difficult to spread uniformly
1 point: cosmetic composition is highly difficult to take out from the container and difficult to spread uniformly

An average score of the professional panelists of not less than 4 was marked with ⊙, not less than 3 and less than 4 was marked with ○, not less than 2 and less than 3 was marked with Δ, and less than 2 was marked with x.

### (Evaluation criteria of affinity to the skin)

When a cosmetic composition is spread on the back of a hand,
5 points: affinity to the skin is very good
4 points: affinity to the skin if good
3 points: normal
2 points: bad affinity to the skin
1 point: very bad affinity to the skin

An average score of the professional panelists of not less than 4 was marked with ⊙, not less than 3 and less than 4 was marked with ○, not less than 2 and less than 3 was marked with Δ, and less than 2 was marked with x.

The results are shown in Table 1.

**Table 1**

| | | Ex. 1 | Ex. 2 | Ex. 3 | Ex. 4 | Comp. Ex. 1 | Comp. Ex. 2 | Comp. Ex. 3 | Comp. Ex. 4 |
|---|---|---|---|---|---|---|---|---|---|
| basic amino acid derivative | Compound of Prod. Ex. 1 | 1 | 5 | - | - | - | - | - | - |
| | Compound of Prod. Ex. 2 | - | - | 1 | - | - | - | - | - |
| | Compound of Prod. Ex. 3 | - | - | - | 1 | - | - | - | - |
| | Compound of Prod. Ex. 4 | - | - | - | - | - | 1 | 5 | - |
| | Compound of Prod. Ex. 5 | - | - | - | - | - | - | - | 1 |
| additive | 1,3-BG | 5 | 5 | 5 | 5 | 5 | 5 | 5 | 5 |
| | glycerin | 5 | 5 | 5 | 5 | 5 | 5 | 5 | 5 |
| | ethanol | 3 | 3 | 3 | 3 | 3 | 3 | 3 | 3 |
| | methylparaben | 0.1 | 0.1 | 0.1 | 0.1 | 0.1 | 0.1 | 0.1 | 0.1 |
| | phenoxyethanol | 0.5 | 0.5 | 0.5 | 0.5 | 0.5 | 0.5 | 0.5 | 0.5 |
| pH adjuster | citric acid | q.s. | q.s. | q.s. | q.s. | q.s. | q.s. | q.s. | q.s. |
| | water | balance | balance | balance | balance | balance | balance | balance | balance |
| | total | 100 | 100 | 100 | 100 | 100 | 100 | 100 | 100 |
| | | | | | | | | | |
| evaluation | Stability of composition | ⊙ | ⊙ | ⊙ | ⊙ | ⊙ | × | × | × |
| | coatability | ⊙ | ○ | ⊙ | ⊙ | ○ | Δ | × | Δ |
| | affinity to skin | ⊙ | ⊙ | ⊙ | ⊙ | × | ⊙ | ○ | ○ |

| | | | | | | | | | |
|---|---|---|---|---|---|---|---|---|---|
| 1,3-BG: 1,3-butylene glycol | | | | | | | | | |

The cosmetic composition free of a medium-chain acyl basic amino acid derivative (Comparative Example 1) showed good stability but showed very bad affinity to the skin. The cosmetic compositions containing a lauroyl amino acid derivative (Comparative Examples 2 - 4) showed comparatively good affinity to the skin but showed bad stability due to gelation and were difficult to apply.

On the other hand, the cosmetic compositions containing the medium-chain acyl basic amino acid derivative of the present invention (Examples 1 - 4) were stable, easily applied, and very superior in the affinity to the skin.

### [Industrial Applicability]

The present invention can provide a medium-chain acyl basic amino acid derivative which, when blended in a liquid cosmetic such as skin lotion, skin milk and the like, suppresses gelation while maintaining affinity to the skin and the like and can afford a stable preparation.

## Claims

1. A compound represented by the formula (1): 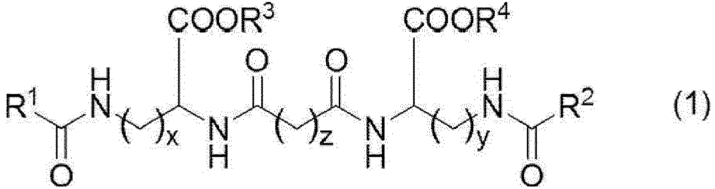 wherein
R¹ and R² are each independently an alkyl group having 5 - 7 carbon atoms or an alkenyl group having 5 - 7 carbon atoms,
R³ and R⁴ are each a hydrogen atom,
z is 7 or 8, and
x and y are each independently an integer of 2 - 4,
or a salt thereof.

2. The compound according to claim 1, wherein x and y are each 4, or a salt thereof.

3. The compound according to any one of claims 1 or 2, wherein R¹ and R² are each independently an alkyl group having 5 - 7 carbon atoms, or a salt thereof.

4. A compound according to claim 1, wherein the compound is selected from bis(N^{ε}-octanoyl-L-lysine)sebacoylamide, bis(N^{ε}-hexanoyl-L-lysine)azelaoylamide, and bis(N^{ε}-octanoyl-L-lysine)azelaoylamide, or a salt thereof.

5. A cosmetic composition comprising at least one kind from the compound according to any one of claims 1 to 4 and a salt thereof.

## Patentansprüche

1. Verbindung der Formel (1): 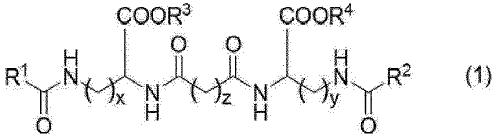 worin
R¹ und R² jeweils unabhängig eine Alkylgruppe mit 5 bis 7 Kohlenstoffatomen oder eine Alkenylgruppe mit 5 bis 7 Kohlenstoffatomen sind,
R³ und R⁴ jeweils ein Wasserstoffatom sind,
z = 7 oder 8 ist und
x und y jeweils unabhängig eine ganze Zahl von 2 bis 4 sind,
oder ein Salz davon.

2. Verbindung nach Anspruch 1, worin x und y jeweils 4 sind, oder ein Salz davon.

3. Verbindung nach einem der Ansprüche 1 oder 2,
worin R¹ und R² jeweils unabhängig eine Alkylgruppe mit 5 bis 7 Kohlenstoffatomen sind, oder ein Salz davon.

4. Verbindung nach Anspruch 1, wobei die Verbindung aus Bis(N^{ε}-octanoyl-L-lysin)-sebacoylamid, Bis(N^{ε}-hexanoyl-L-lysin)azelaoylamid und Bis(N^{ε}-octanoyl-L-lysin)azelaoylamid ausgewählt ist, oder ein Salz davon.

5. Kosmetische Zusammensetzung, die zumindest eine Art einer Verbindung nach einem der Ansprüche 1 bis 4 oder ein Salz davon umfasst.

## Revendications

1. Composé représenté par la formule (1) : 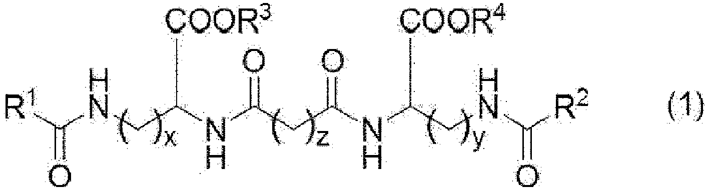 dans lequel
R¹ et R² sont chacun indépendamment un groupe alkyle ayant 5 à 7 atomes de carbone ou un groupe alcényle ayant 5 à 7 atomes de carbone,
R³ et R⁴ sont chacun un atome d'hydrogène,
z est 7 ou 8, et
x et y sont chacun indépendamment un entier de 2 à 4, ou un sel de celui-ci.

2. Composé selon la revendication 1, dans lequel x et y sont chacun 4, ou un sel de celui-ci.

3. Composé selon l'une quelconque des revendications 1 ou 2, dans lequel R¹ et R² sont chacun indépendamment un groupe alkyle ayant 5 à 7 atomes de carbone, ou un sel de celui-ci.

4. Composé selon la revendication 1, dans lequel le composé est choisi parmi le bis (N^{ε}-octanoyl-L-lysine)sébacoylamide, le bis (N^{ε}-hexanoyl-L-lysine)azélaoylamide et le bis (N^{ε}-octanoyl-L-lysine)azélaoylamide, ou un sel de celui-ci.

5. Composition cosmétique comprenant au moins un type du composé selon l'une quelconque des revendications 1 à 4 et un sel de celui-ci.
